# EUROPEAN PATENT APPLICATION

(11) **EP 4 147 730 A1**
(43) Date of publication of application: **15.03.2023**
(21) Application number: 21195817.8
(22) Date of filing: 09.09.2021
(51) Int. Cl.: A61L 27/22, A61L 27/38, A61L 27/50, A61L 27/52

(54) **METHOD OF PREPARING A FUNCTIONAL HYDROGEL MATERIAL THAT CAN BE INJECTED**

(71) Applicant: ETH Zurich, 8092 Zürich (CH)
(72) Inventor: Katzschmann, Robert Kevin, 8044 Zürich (CH); Yasa, Oncay, 4053 Basel (CH); Tiruneh, Fikru Mazengia, 8046 Zürich (CH)

(57) **Abstract**

A method of preparing a functional hydrogel material (9) for injection into a human or animal body is indicated, the method comprising the step of polymerizing a prepolymer solution (1), in order to form the functional hydrogel material (9). Red blood cells (5) of which the cell membranes have been made porous are integrated into the prepolymer solution (1) before and/or during the polymerization. Furthermore, a functional hydrogel material is indicated comprising a hydrogel network formed by a polymer (10). Red blood cells (5) of which the cell membranes have been made porous are integrated in the hydrogel network.

## Description

### TECHNICAL FIELD

The present invention relates to a method of preparing a functional hydrogel material for injection into a human or animal body.

### PRIOR ART

Functional hydrogels are increasingly investigated and used for various biomedical applications and in particular in many fields of medicine, such as tissue engineering and regenerative medicine. Functional hydrogels can for example be used to promote cartilage and bone regeneration. It is also known to use functional hydrogels e.g. in mesenchymal stem cell and chimeric antigen receptor (CAR) T-cell therapies.

Hydrogels are generally gels with a high water content. They comprise hydrophilic but water-insoluble polymers, which can swell considerably in water while largely retaining their shape and structure.

Hydrogels comprise a three-dimensional network of a crosslinked hydrophilic polymer that does not dissolve in water. Due to hydrophilic functional groups that are attached to the polymer main structure, hydrogels have a distinct ability to absorb water. At the same time, the cross-links between the polymeric network chains prevent the dissolution of the hydrogel. Therefore, a hydrogel can swell to a considerable volume without losing its material cohesion. Depending on the properties of the polymer used and the type and density of the network links, hydrogels can bind different amounts of water. They also have a high degree of flexibility and elasticity, which, due to their high water content, is very similar to biological tissues.

Due to their special properties, hydrogels are for example used in wound care. They are particularly suitable for moisturizing or rehydrating dry wounds. In itchy skin conditions, they provide relief through their cooling effect.

Depending on the indication, a hydrogel may also contain certain active ingredients to accelerate the healing process of a wound on the body surface or inside the body.

A new field where hydrogels can also be used is soft robotics, which is a rapidly emerging subfield of robotics that relies on soft and flexible materials, such as hydrogels, to enable new functionalities. Since soft robots are not using rigid links and joints, they are considered to be safer for human-robot interactions than conventional rigid-bodied robots. Small-scale soft robots can also be applied for carrying out various tasks within the human or animal body. To navigate a soft robot to a desired position within the body, magnetic particles can for example be integrated in the hydrogel that forms the soft robot, and the soft robot can then be actuated and moved by means of applying an external magnetic field. To track the movements and visualize the operation of the robot within the body, an appropriate imaging method can be employed, such as magnetic resonance imaging (MRI) and ultrasound imaging.

Small-scale soft robots have the potential to substantially impact the way diseases such as cancer can be treated. Existing treatment methods are typically affecting not only the cancerous tissue, but also other healthy cells in the body. Other applications of such an injectable robot are the controlled deployment of drugs to target sites within the body.

Multiple challenges, however, still need to be addressed before functional hydrogels and particularly soft robots can be widely used in daily clinical routine. One of these challenges is the insertion of the functional hydrogels into the human or animal body.

One proposed solution commonly used in tissue engineering is the injection of non-polymerized prepolymer solutions to the desired sites of actions, followed by the induction of the polymerization reaction (chemical crosslinking) of the molecular building blocks in situ. However, this technique has several disadvantages and is not applicable for most (bio)materials. First, it is not possible to accurately determine the outcome of the polymerization reaction at the desired site of action. Second, it is difficult to crosslink materials by means of a photopolymerization reaction deep inside of the body. Third, most of the photoinitiators are toxic to the human body and their administration for such an application can result in undesired side effects.

Therefore, it is safer and more advantageous to produce the functional hydrogels outside of the body and to then deploy them in the body. Due to the advantages of minimally invasive operations, there is a demand for functional hydrogels to be injectable e.g. through a cannula, a tube or a catheter. When deploying functional hydrogels and in particular small-scale soft robots e.g. for operations in the human body, injectability is one of the first encountered challenges. Neglecting this issue can lead to a functional hydrogel being blocked in the cannula or even destroyed during injection. Furthermore, it needs to be ensured that the structure remains intact and that no damages occur to the hydrogel during the injection. A further requirement, in order to avoid immunogenic reactions, is the biocompatibility, which limits the materials and coatings that can be used for the hydrogels.

Certain preformed cryogel scaffolds have already been found to be compatible with conventional cannula-syringe injections. Due to their shape-memory properties, a good shape recovery could be observed, as long as the inner diameter of the cannula is not too small with respect to the cryogel scaffold.

Cryogels are formed by freezing prepolymer solutions at temperatures of e.g. -20°C for around 30 hours. During this time, water droplets form into small ice crystals and the polymer gelates around them. Once the hydrogel is fully polymerized, the water crystals can be thawed, which then leaves a gel with micro-scale interconnected cavities. Thus, due to the required freezing and thawing steps, the production of cryogels takes a relatively long time.

An example of an injectable frozen gel with a shape memory function is disclosed in CN 110256856 A.

Further documents which disclose injectable scaffolds made of a cryogel are for example WO 2012/149358 A1 and US 2014/0227327 A1.

Furthermore, CN 109701073 A discloses an injectable cartilage repairing hydrogel and CN 108853598 A an autologous bioprotein hydrogel for retina surgery. WO 2015/188064 A1 proposes a peptide hydrogel that can be used for a variety of applications.

CN 11128214 A discloses a contact lens made from a hydrogel that is based on erythrocyte membranes. Amino components of the erythrocyte cell membrane protein are used for producing the hydrogel.

Besides, in the journal article *Soft erythrocyte-based bacterial microswimmers for cargo delivery;* Y Alapan, O Yasa, O Schauer, J Giltinan, AF Tabak, V Sourjik, M Sitti; Science Robotics 3 (17), eaar442, it is proposed to attach erythrocytes to Escherichia coli bacteria, in order to form a microswimmer that can be used to transport drugs as well as iron oxide nanoparticles to a desired position within the body. By means of a hypotonic treatment, pores in the cell membrane of the erythrocyte are opened, in order to receive the drug or iron oxide particles, which is followed by an isotonic treatment for closing the pores and, thus, encapsulating the particles within the erythrocyte.

A particular challenge with magnetic particles used for navigation is that they are often cytotoxic. As a consequence, complications due to the immune system of the body are likely to occur. Coating of these particles with a biocompatible material has been proposed to avoid such problems. However, since the coating can degenerate over time, this is only a short-term solution.

### SUMMARY OF THE INVENTION

It is an object of the present invention to provide a method of preparing a functional hydrogel material that can be injected into a human or animal body through a small cannula, tube or catheter without being damaged.

This object is solved by the method as claimed in claim 1. Further embodiments of the method are provided in dependent claims 2 to 14. A functional hydrogel material is claimed in claim 15.

The present invention thus provides a method of preparing a functional hydrogel material for injection into a human or animal body, the method comprising the step of polymerizing a prepolymer solution, in order to form the functional hydrogel material. According to the invention, red blood cells of which the cell membranes have been made porous are integrated into the prepolymer solution before and/or during the polymerization.

Thus, red blood cells are integrated into the prepolymer solution, which have been treated, preferably treated in a targeted manner for this particular purpose, to be porous in a previous method step. The red blood cells that are integrated into the prepolymer solution thus usually have a more porous membrane than in their natural form.

By including red blood cells of which the cell membranes have been made porous into the prepolymer solution and, thus, into the hydrogel material, the functional hydrogel material becomes particularly compressible. The pores allow the cell content to diffuse out, and liquid, such as water and/or phosphate-buffered saline (PBS) to enter the cell. Therefore, the red blood cells used in the method preferably have a reduced hemoglobin-content. More preferably, they are substantially free of hemoglobin. Thus, through their open pores, the red blood cells can absorb liquid, such as water. When compressing the hydrogel material on a macroscopic dimension, the swollen red blood cells are compressed too and they release the previously stored liquid. This causes them to shrink in size and allows the hydrogel material to deform on a macroscopic level, in order to pass through a small cannula, tube, or catheter without being damaged. Once injected, the deformed red blood cells reabsorb liquid and swell to their initial size. This causes the hydrogel material to regain its initial shape. Therefore, in summary, the red blood cells act like sponges that swell when absorbing liquid and shrink by liberating liquid when compressed.

In the hydrogel, the red blood cells preferably act as "space-holders", so that the polymerization occurs around them and traps them in the final hydrogel network formed by the polymer. The red blood cells are preferably of natural origin, but could in principle also be artificially produced.

By being able to pass through a small cannula, tube, or catheter, the hydrogel material is also able to pass through particularly narrow channels or vessels within the human or animal body.

Thus, use is made of the inherent advantage of red blood cells that they are robust to mechanical stress and can keep their shape after temporary deformation, e.g. while passing through a narrow capillary. Thus, the porous red blood cells give the hydrogel material large deformation abilities and allows the hydrogel to pass through cannulas that are by an integer multiple smaller than the structure formed by the hydrogel. Once the hydrogel has been injected, the red blood cells regain its original size due to diffusion of water into the red blood cell. Thus, except that they have been made porous, the red blood cells contained in the prepared hydrogel are preferably still intact. Another advantage of red blood cells is of course their excellent biocompatibility.

The porous red blood cells can also be used as carriers of various functional substances such as drugs, proteins, cells, or magnetic particles. The pores of the red blood cells can be such that the functional substance(s) are trapped within the cell, while still enabling liquids, in particular water, to pass. Of course, it is also possible to integrate the functional substance(s) outside of the red blood cells in the hydrogel material or to integrate the functional substance(s) both inside and outside of the red blood cells in the hydrogel material.

Due to its pronounced deformability, it is preferably not only possible to inject the functional hydrogel material into the body, but also to retract it from the body through a small cannula, tube, or catheter without being damaged. This enables the products that have been administered to be retracted after they have completed their function inside the body or if they create any unwanted side effects. In this way, it is for example possible to avoid any cytotoxic side effects due to magnetic particles that are integrated in the hydrogel for navigation purposes.

A functional hydrogel material is generally a hydrogel material that is adapted to perform a certain function, i.e. to have a certain effect, within the human or animal body. The function can for example be due to a drug or due to cells, in particular stem cells, carried by the hydrogel. The function can also be related to the tissue-like properties of the hydrogel, i.e. the hydrogel can be used to replace or supplement a certain tissue of the body.

The functional hydrogel can for example be used in mesenchymal stem cell and chimeric antigen receptor (CAR) T-cell therapies. The functional hydrogel can also form a soft robot for controlled therapeutics delivery operations. A possible field of application is particularly cancer treatment.

The prepolymer solution contains one or several prepolymers that are capable of polymerizing into a polymer network, in order to form the main structure of the hydrogel material.

The step of polymerizing the prepolymer solution, in order to form the functional hydrogel material, can be carried out in various ways, depending on the type of prepolymer solution. For example, photopolymerization by means of visible light or by means of ultraviolet (UV)-exposure can be applied and/or the polymerization can be effected by means of heat and/or by means of another chemical substance.

The polymer and the red blood cells of the hydrogel material prepared by the method as indicated, preferably form a scaffold having a well-defined three-dimensional outer shape. The prepared hydrogel material can particularly form a basic three-dimensional geometric body, such as a cube, a cuboid, a prism, a pyramid, a sphere, a cylinder, or a cone. A mold can be used, in order to achieve such or other structures. The use of a 3D bioprinter is also conceivable to fabricate the hydrogel scaffold.

Within the prepared hydrogel material, the red blood cells are preferably easily visible to the human eye with the aid of a microscope. Thus, it is preferably possible to identify the individual red blood cells within the hydrogel material and advantageously to also recognize the characteristic outer shape of the red blood cells which is preferably similar or almost the same as the one of natural, untreated red blood cells, i.e. the shape of a disc dented in the middle.

Preferably, before being integrated in the prepolymer solution, the red blood cells used for preparing the functional hydrogel material have been subjected to a special treatment, in order make their cell membranes porous. Thus, the cell membranes have advantageously been made porous in a targeted way with regard to the later use of the red blood cells in the preparation of the hydrogel material. The red blood cells that are integrated in the polymer solution are particularly more porous than they normally are in nature. Preferably, the red blood cells are uniformly distributed within the entire prepared hydrogel material.

In order to make their cell membranes porous, the red blood cells are preferably exposed to a hypotonic treatment and advantageously to a subsequent isotonic treatment. The step of the hypotonic treatment is preferably carried out before the red blood cells are integrated into the prepolymer solution. There are various other ways to make the cell membranes porous, but the hypotonic treatment has turned out to be particularly efficient.

Preferably, red blood cells are used of which the cytoplasm has been reduced by at least 50 wt%, more preferably by at least 75%. In this way, a good porosity can be reached.

In a particularly preferred embodiment, the prepolymer solution contains gelatin methacryloyl (GeIMA). GelMA is a versatile material that is well suited for a wide range of biomedical applications. GelMA forms a crosslinked polymer when exposed to light irradiation and has good mechanical properties, very close to those of natural tissues. The physical characteristics of GelMA, in particular the flexibility and elasticity, are adjustable, in order to form a specifically tailored functional hydrogel material. Most importantly, however, it has turned out that combining GelMA with the porous red blood cells leads to a hydrogel material with a particularly high deformability, such that it can be injected through small cannulas without altering its structure and without being damaged. The use of other prepolymer materials that are suitable to form a hydrogel material is of course possible, such as sodium alginate, collagen, chitosan, or agarose.

In particular when using gelatin methacryloyl as the prepolymer, the concentration of the prepolymer in the prepolymer solution is preferably between 2 wt% and 4wt%, more preferably between 2.5 wt% and 3.5 wt%, in particular about 3 wt%. With such a concentration of the prepolymer, in particular of GelMA, hydrogel materials can be fabricated having optimal mechanic properties in particular as concerns the injectability of the material through small cannulas.

The number of the porous red blood cells in the prepolymer solution is preferably between 7.5 and 10 billion/mL, more preferably between 8.5 and 9.5 billion/mL, most preferably between 8.7 and 9 billion/mL. Higher concentrations of red blood cells result in more deformable hydrogel scaffolds, but their structural integrity is not guaranteed, since polymerization cannot occur properly. However, hydrogel scaffolds with a lower concentration of red blood cells have a better structural integrity, but are less deformable because their stiffness is increased by the polymer. Therefore, an optimal ratio of the polymer and the red blood cells in the hydrogel material is achieved with the concentrations as indicated.

The concentrations as indicated above with respect to the prepolymer and the red blood cells are particularly optimal in combination and in particular if GelMA is used as the prepolymer. By tuning the concentration of the porous red blood cells inside the prepolymer solution, the compression and deformation ability of the final hydrogel can be adjusted.

The pores of the cell membranes of the red blood cells preferably have a diameter between 15 nm and 400 nm, more preferably between 50 nm and 150 nm. Advantageously, more than 90%, more advantageously more than 95%, of the pores have a diameter in this range. In this respect, a pore is regarded to be an opening in the cell membrane of the red blood cell that has been formed by a certain treatment for making the blood cells porous.

The prepolymer solution is preferably polymerized in a mold. In this way, a well-defined three-dimensional outer shape can be given to the hydrogel. The mold can be fabricated by means of additive manufacturing.

The functional hydrogel material prepared by the method as indicated is preferably placed within an injection device, which serves to inject or retract the functional hydrogel material into or from the human or animal body. The injection device can for example be in the form of a syringe and preferably comprises a cannula, a tube and/or a catheter for injecting or retracting the functional hydrogel material.

After placing the functional hydrogel material within the injection device, the functional hydrogel material can be injected into the human or animal body through the cannula, the tube and/or the catheter. The injection is preferably a minimally invasive injection that can for example be intravenous, subcutaneous or intraocular.

Alternatively, the functional hydrogel material can also be placed within the injection device by means of retracting the functional hydrogel material from the body of the human or the animal through the cannula, the tube and/or the catheter.

Depending on the application, the injection or retraction of the hydrogel material can be carried out by e.g. a physician, a nurse or by the patient himself.

If the red blood cells contained in the functional hydrogel material originate from the same human or animal, in which the functional hydrogel material is to be injected or from which the functional hydrogel material has been retracted, an optimal biocompatibility can be achieved.

On the other hand, if the red blood cells contained in the functional hydrogel material originate from a different human or animal, than the human or animal in which the functional hydrogel material is to be injected or from which the functional hydrogel material has been retracted, then their procurement might become easier with less risk for the patient. Also, red blood cells from certain animals could be used, in order to make use of their different sizes, which when integrated into a single hydrogel scaffold result in different mechanical properties. For example, depending on the application, the entire range of red blood cells having a diameter of 2.2 µm as present in mouse-deer up to red blood cells with a diameter of 50 µm as found in giant salamanders or even combinations of red blood cells originating from different species could be used.

The functional hydrogel material preferably has a structure which, in a relaxed state, covers a plane area that has a larger inner maximum diameter than the diameter of an inner lumen of the cannula, the tube and/or the catheter of the injection device. The inner maximum diameter represents the diameter of the largest circle that can be arranged completely within the hydrogel material. More preferably, the inner maximum diameter of the hydrogel is by an integer multiple larger, even more preferably more than 5 times larger or even more than 10 times larger, than the diameter of an inner lumen of the cannula, the tube and/or the catheter of the injection device.

Furthermore, the invention relates to a functional hydrogel material for injection into a human or animal body, produced in particular by the method as indicated. The functional hydrogel material comprises a hydrogel network formed by a polymer. Red blood cells of which the cell membranes have been made porous are integrated in the hydrogel network.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: schematically shows the different steps of the inventive method of preparing an injectable hydrogel material scaffold according to a preferred embodiment;
- Fig. 2: schematically shows the inventive functional hydrogel material before, during and after compression/deformation;
- Fig. 3: schematically shows the inventive functional hydrogel material, in which magnetic particles and a drug have been integrated;
- Fig. 4a: shows an inverted microscope image of a functional hydrogel material which has been fabricated according to the inventive method;
- Fig. 4b: shows a scanning electron microscope image of the same hydrogel material as in Fig. 4a; and
- Fig. 4c: shows a scanning electron microscope image of the same hydrogel material as in Fig. 4a, but with an increased magnification.

### DESCRIPTION OF PREFERRED EMBODIMENTS

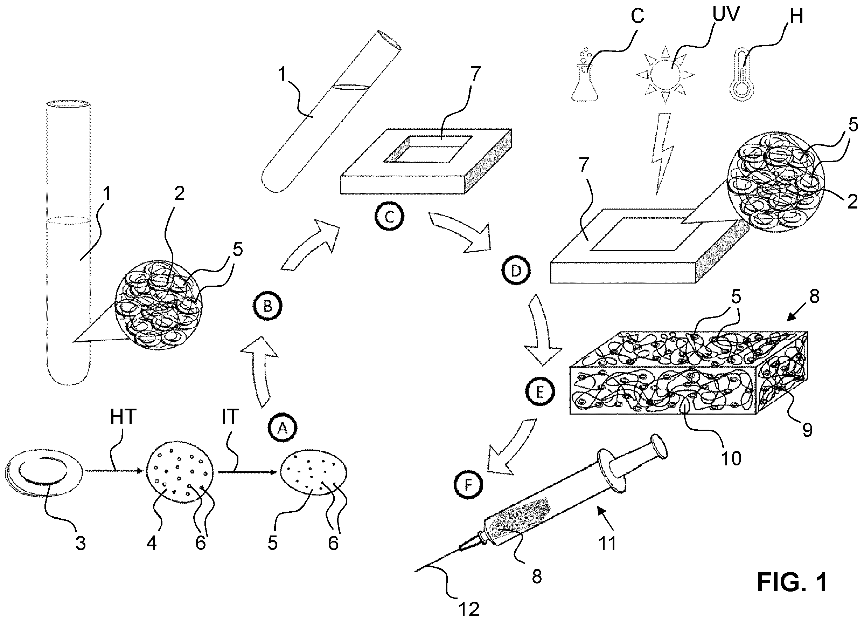

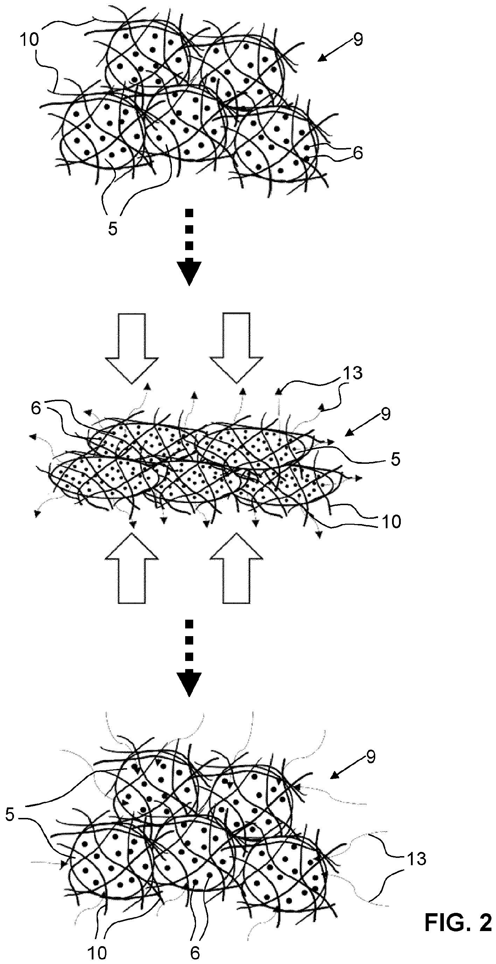

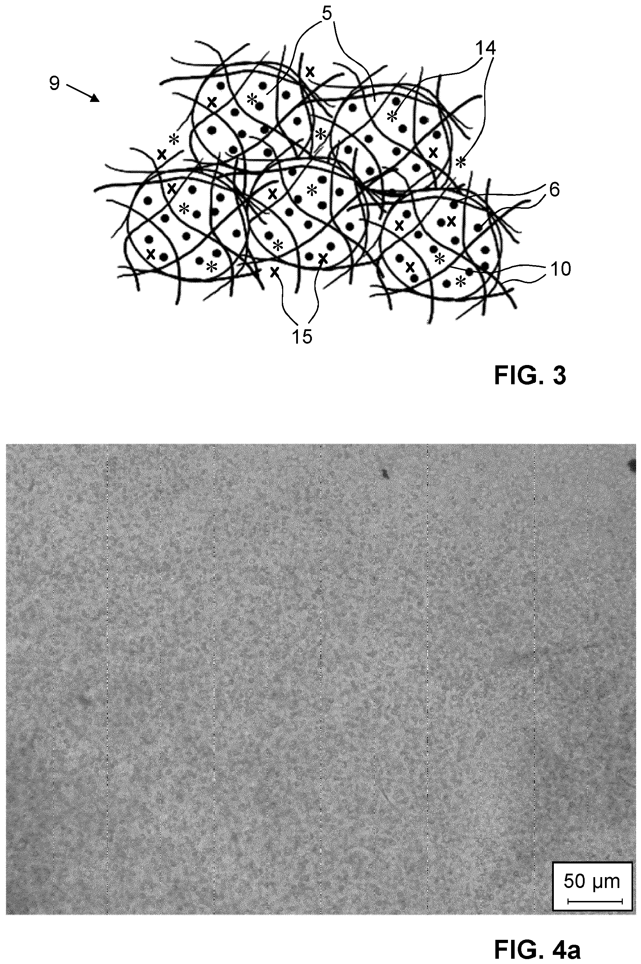

Figure 1 schematically shows the different steps A to F of the inventive method of preparing an injectable hydrogel material scaffold 8 according to a preferred embodiment. Figure 2 gives an insight into the hydrogel material 9 that forms the such prepared scaffold 8, in order to explain its particular compression and deformation capabilities. In figure 3, an inventive hydrogel material 9 is schematically illustrated that comprises magnetic particles and a drug integrated within the material.

For preparing an inventive injectable hydrogel material scaffold 8, as shown in Figure 1, red blood cells 3 are made porous in a first step A. For this purpose, the red blood cells 3 are subjected to a hypotonic treatment HT. The hypotonic treatment HT causes an absorption of liquid into the red blood cells 3 and, as a result, a swelling of the red blood cells 3. As a consequence, swollen red blood cells 4 are obtained. Due to the swelling, a stretching of the membrane of the red blood cells 4 occurs, until pores of around 100 nm are formed. Thus, pores 6 are formed in the membrane of the swollen red blood cells 4. Furthermore, when incubating the red blood cells 3 in a hypotonic solution, the intracellular content, i.e. the cytoplasm, of the swollen red blood cells 4 (mainly hemoglobin) diffuses out of the cells through the opened pores. On the other hand, the pores 6 can be used as entry points to include e.g. magnetic particles, drugs, cells and/or proteins in the swollen red blood cell 4. By means of a subsequent isotonic treatment IT, the pores 6 are partially resealed, in order to trap the magnetic particles, drugs, cells and/or proteins within the resulting porous red blood cell 5 and to avoid further growth as well as an eventual bursting of the red blood cells. However, a free exchange of liquid, in particular of water or phosphate-buffered saline (PBS), through the pores 6 into and out of the red blood cells 5 is still possible. Due to this freely possible liquid exchange, the porous red blood cells 5 have the advantage of higher compressibility and deformability, since a larger volume can be squeezed upon stress.

The red blood cells 5 of which the cell membranes have been made porous in step A are then mixed with a prepolymer 2, in order to form a prepolymer solution 1 (step B in Figure 1). The prepolymer 2 can particularly be gelatin methacryloyl (GelMA).

In the subsequent step C, the prepolymer solution 1 is brought into a mold 7, which can particularly be a 3D-printed mold. The mold 7 defines the final size and shape of the injectable hydrogel material scaffold 8.

With the polymer solution 1 being in the mold 7, a treatment is carried out in step D, in order to polymerize the polymer solution 1 and to form the functional hydrogel material 9. During this process, the prepolymer 2 is crosslinked, in order to form a polymer 10. In the case that GelMA is used as the prepolymer 2, the treatment preferably comprises an ultraviolet (UV)-exposure UV. For this purpose, UV-light having a wavelength of approximately 365 nm is preferably used. Depending on the choice of the prepolymer 2, however, other treatments for polymerizing the polymer solution 1 are conceivable alternatively or in addition, such as a chemical treatment C and/or a heat treatment H.

Next, the polymerized hydrogel material 9 is demolded from the mold 7 (step E in Figure 1). It is then in the form of the injectable hydrogel material scaffold 8 which has a well-defined outer shape as given by the mold 7. The outer shape of the injectable hydrogel material scaffold 8 can for example be cuboid as shown in Figure 1.

Finally, the such prepared hydrogel material scaffold 8 is inserted in an injection device, such as a syringe 11, in order to be injected trough a cannula 12 of the syringe 11 into a human or animal body.

Due to the presence of the porous red blood cells 5 in the hydrogel material 9, the hydrogel material scaffold 8 is deformable and compressible to such a high degree that it fits through the inner lumen of the cannula 12 without being damaged. Experiments have shown that it is even possible to retract the hydrogel material scaffold 8 from the human or animal body through the cannula 12 and into the syringe 11.

The deformation and compression properties of the hydrogel material 9 are explained in the following with reference to Figure 3: In the relaxed state of the hydrogel material 9, e.g. before injection when being inside of the syringe 11, the crosslinked polymer 10 forms a network in which the porous red blood cells 5 are entrapped. The porous red blood cells 5 are also in a relaxed state and filled with liquid, such as PBS. This state of the hydrogel material 9 is shown in the top part of Figure 3.

When the hydrogel material 9 is injected through the cannula 12 of a syringe, the material is deformed and compressed and so are the porous red blood cells 5, as shown in the middle part of Figure 3. Due to the porosity of the cell membrane of the red blood cells 5, liquid 13 that has previously been stored inside of the red blood cells is allowed to be expelled from the porous red blood cells 5. In this way, the porous red blood cells 5 and, thus, the hydrogel material 9, can be compressed fast and to a considerable degree without getting damaged.

After being injected, i.e. after passing through the cannula 12, the porous red blood cells are relaxed again, meaning that the liquid 13 is sucked back through the porous cell membranes into the cells (lower part of Figure 3). The hydrogel material 9 is in the same state again as before the injection. Due to the distinct compressibility of the porous red blood cells 5, the hydrogel material 9 is able to pass, without being damaged, through particularly small openings, such as the one of the cannula 12, and to again adopt its original structure and shape after passing through.

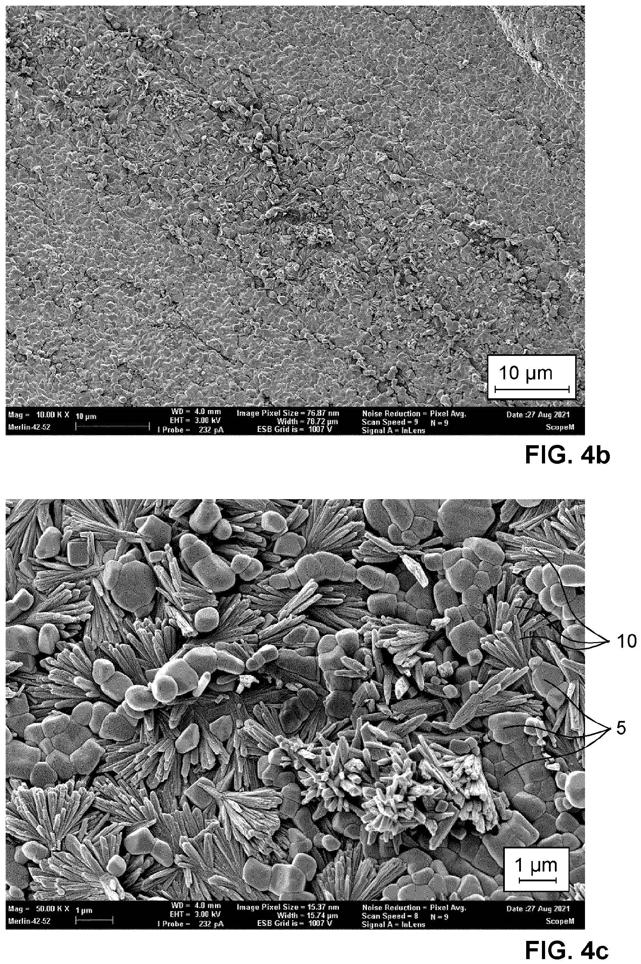

Figure 4 schematically illustrates an inventive hydrogel material 9 comprising magnetic particles 14 and a drug 15 integrated in the hydrogel material 9. Alternatively or additionally, reference numerals 14 and 15 could also refer to e.g. proteins of cells, in particular stem cells. The particles 14 and the drug 15 are preferably located within the porous red blood cells 5 and, more preferably, trapped there due to the lower size of the pores 6. Alternatively or additionally, the particles 14 and the drug 15 can also be present outside of the porous red blood cells 5, as shown in Figure 4.

### Experiment

The main materials used for fabricating a hydrogel material 9 in a concrete experiment were porous red blood cells 5, gelatin methacryloyl (GelMA), lithium phenyl-2,4,6-trimethylbenzoylphosphinate (LAP) and barium ferrite (BaFe₁₂O₁₉) magnetic microparticles (MMPs). Purified mouse red blood cells were purchased from Innovative Research (Novi, MI). LAP and GelMA with gel strength of 300 g Bloom and 40% degree of substitution were purchased from Sigma-Aldrich (St. Louis, MO). MMPs with a diameter of 4-5 µm were acquired from Hoosier Magnetics Inc. (Ogdensburg, NY).

The fabrication of the desired small-scale magnetic soft robots can be summarized by the following main steps:
- washing of the red blood cells with 1x phosphate-buffered saline (PBS) followed by the hypotonic-isotonic treatment;
- preparation of the prepolymer solution and shaping of the hydrogel material scaffold in the form of small-scale soft robots through photoinitiated radical polymerization reaction in negative molds; and
- magnetization of the small-scale soft robots.

In the following, the procedure used to fabricate the soft robots is described in more detail:
The procedure starts with the preparation of the porous red blood cells:
In the first step, the purified RBCs are washed three times with 1x PBS. Briefly, 500 µL or red blood cells are diluted with 4:5ml 1x PBS. This solution is centrifuged for 5 minutes at 2000 g and at 4 °C, using a Centrifuge 5702 R (Eppendorf, Hamburg, Germany). The supernatant is then discarded and the pellet is dissolved to a total of 5mL PBS. After repeating these steps three times, the washed red blood cells are stored in a fridge at 4 °C until further usage.

In the next step, the red blood cells are treated with a hypotonic solution. The washed red blood cells are then centrifuged for 5 minutes at 2000 g and at 4 °C to discard the supernatant. The pellet is dissolved in 1mL of (1:1)(PBS:dH₂O) and pipetted as well as vortexed (Vortex-Genie 2, Scientific Industries, New York, USA) for a proper mixing. The solution is then incubated in an ice bath on a Rotamax 120 (Heidolph, Schwabach, Germany) at 200 rpm for 15 minutes. The treated solution is then centrifuged for 5 minutes at 2000 g and at 4 °C to discard the supernatant. Finally, the treated pellet is dissolved in 1mL PBS, pipetted, vortexed, and stored in the fridge at 4 °C until further usage.

The subsequent preparation and polymerization of the prepolymer solution is carried out as follows:
The prepolymer solution is prepared in a separate tube. 67.5 mg (3 wt%) of GelMA are weighed using a Genius ME 235s scale (Sartorius, Göttingen, Germany) and dissolved in 1.8 mL of PBS. The solution is vortexed and sonicated at 65 °C using a USC-TH Ultrasonic Cleaner (VWR, Radnor-Pennsylvania, PA) until GelMA is completely dissolved in PBS. Next, 67.5 mg (3 wt%) of LAP and 45 mg (2 wt%) MMPs are weighed and added to the dissolved GelMA in the dark. From this point on, it is important not to expose the prepolymer solution to light, since polymerization can already begin at visible light. To protect the prepolymer solution, aluminium foil is wrapped around the tube. The prepolymer solution is then vortexed and sonicated at 65 °C until the LAP is dissolved too. The prepolymer solution is then added to the pellet of red blood cells and only vortexed, since sonication destroys the red blood cells.

In a further step, a negative mold is 3D-printed (Ultimaker S5 Pro, Ultimaker, Utrecht, Netherlands) out of polylactic acid (PLA) and cleaned with 70 % (v/v) ethanol. When pouring the prepolymer solution into these molds, air bubbles must be avoided. This can be done by not pushing all the prepolymer solution out of the pipette tip, but leaving a small amount in the tip. The molds are then exposed to UV-radiation (Form Cure, Formlabs, Somerville, MA) three times for 2 minutes to physically crosslink the prepolymer solution. Once cut out of the molds with a scalpel, the hydrogels are ready to be magnetized and injected through a conventional syringe system.

The magnetization profile is programmed as follows:
In a last step, a magnetization profile needs to be established over the small-scale soft robots in order to actuate them with an externally-applied homogeneous magnetic field. This can be completed by wrapping the fabricated small-scale soft robots on a cylinder and exposing them to a magnetic field that will be higher than the coercivity of the integrated ferromagnetic microparticles.

### Injectability of the experimentally prepared hydrogel

To inject the prepared soft robots, conventional three-part needle-syringe systems are used. The syringes have a centric cone with a Luer-Lock connector and no inner coating. Syringes with a volume of 10 mL and 20 mL are used with needle gauge sizes of 14 G, 16 G, 18 G and 20 G. The different needle sizes can be extracted from Table 1. For all the injections, the syringes are filled with approximately 4 mL of PBS. The robots are then carefully loaded onto a spatula and put into the PBS. When emerged into the solution, the robot detaches from the spatula and sinks to the syringe opening. The robots are injected into a beaker filled with PBS to at least 3/4 of its capacity. For injections through 14 G and 16 G needles, a constant pressure can be applied on the plumber. However, for injections through 18 G and 20 G an in-and-out motion needs to be applied.

**Table 1: Overview of different needle gauges.**

| Gauge | Inner Ø (mm) | Outer Ø (mm) |
|---|---|---|
| 14 | 1.753 | 2.108 |
| 18 | 1.346 | 1.651 |
| 20 | 0.965 | 1.245 |
| 22 | 0.635 | 0.914 |

### Optimization of the prepolymer and red blood cells concentration

The optimal ratio of prepolymer wt%, in this case GelMA, and number of RBCs/mL were investigated. This ratio dictates the mechanical properties of the fabricated hydrogels; the polymer gives the hydrogel its structure and the red blood cells give the hydrogel its deformability. If the polymer wt% is too high, the resulting hydrogels are too stiff to pass through a needle, but if the prepolymer wt% is too low, the prepolymer solution does not polymerize enough and remains in a semi-liquid state. Analogously, a prepolymer solution with a too high amount of red blood cells/mL does not fully polymerize and with a too little amount of red blood cells/mL, it does not give the hydrogel enough flexibility to pass through the needles (i.e. cannulas).

The optimal ratio has been determined by iterating through the above-mentioned parameters and injecting the resulting hydrogels through a 14 G needle. All the injections were recorded and conclusions for the next iteration were drawn from these recordings. The main issues were hydrogels that remained in a liquid state and hydrogels that did not polymerize into a thorough structure. In the end, it was found that concentrations between 2 wt% and 4 wt%, in particular of approximately 3 wt%, of GelMA and between 7.5 and 10 billion/mL, in particular of approximately 8.88 billion red blood cells/mL, are optimal, because hydrogels fabricated with these concentrations can be injected through a 14 G needle without being damaged at all. The subsequent experiments were carried out with these parameters.

### Test of the GelMA control samples

To validate the hypothesis, whether the red blood cells give the fabricated hydrogel scaffold its deformation abilities, a hydrogel scaffold comprising 3 wt% GelMA and no red blood cells was fabricated. This control sample was injected through a conventional needle-syringe system, with a needle size of 14 G. These hydrogel scaffolds always broke and could not recover their initial shape, in contrast to the hydrogel containing porous red blood cells. Therefore, it is assumed that the hypothesis is validated and that the porous red blood cells give the hydrogel scaffolds their deformation abilities.

### Polymerization Reaction Conditions

In order to polymerize to a crosslinked network, the prepolymer solution prepared as indicated above is preferably exposed to UV-radiation of 365 nm wavelength. In the beginning, a self-built UV chamber was used. It comprises a box assembled from kanya profiles, with faces covered by acrylic glass and a UV paint curing lamp attached to the ceiling of the box. The molds, filled with prepolymer solution, were placed on a platform at a distance of approximately 20 cm. An issue with this setup was that as a result of the heat produced by the lamp; the water in the prepolymer solution would slowly condensate, which could lead to dried out hydrogels. To mitigate this problem, it was tried to do a few short cycles of 20-30 seconds or to add a droplet of PBS on the surface of the hydrogel. However, the resulting hydrogels were not consistent; the same cycle times and number of cycles gave different results. Therefore, the UV chamber from Formlabs Inc. (Somerville, MA) was tested. It produces less heat and has a turning table, which improved the polymerization process. Good and consistent results were obtained with three cycles of two minutes in this chamber.

### Injectability through different needle sizes

In the state of the art, injections of hydrogels or cryogels are usually performed through 16 G needles. For the testing the injectability of the hydrogel scaffolds prepared as indicated above, 14 G, 18 G and 20 G needles were used. Table 2 illustrates the results of the injection depending on the size of the (cuboid) scaffold and the needle. It can be observed that the biggest injectable hydrogel dimensions for 14 G, 18 G and 20 G needles are respectively 10x10 mm, 10x10 mm and 5x5 mm. Thus, with the hydrogels prepared as indicated above, a particularly good injectability (ratio of hydrogel scaffold size to needle size) can be achieved. The tested hydrogel scaffold also showed to have a high durability; they could be injected ten times in repetition without any noticeable damage under optical inspection. Furthermore, it has been found that the injectable hydrogels have at least 10 days shelf life when they are stored at 4 °C.

**Table 2: Injectability matrix for different hydrogel and needle sizes.**

| Hydrogel scaffold size (mm) | 14 G | 18 G | 20 G |
|---|---|---|---|
| 10 x 10 x 0.5 | ok | ok | not ok |
| 10 x 5 x 0.5 | ok | ok | not ok |
| 10 x 2.5 x 0.5 | ok | ok | ok |
| 5 x 5 x 0.5 | ok | ok | ok |
| 7 x 2.5 x 0.5 | ok | ok | ok |

### Retraction of the Hydrogels

Once injected into the beaker filled with PBS, the hydrogel could be retracted again with a syringe. It was even possible to retract 10x10x0.5 mm hydrogels with a 14 G needle. It is an effortless process and no precautions need to be taken, only the syringe needs to be placed close enough to the hydrogel. As soon as the plumber of the syringe is pulled, the hydrogel, together with PBS, is sucked into the syringe.

Due to the particularly flexible design of the hydrogel prepared as indicated above, a retraction through the cannula of a syringe is enabled. The same mechanism that allows the hydrogel to pass through the syringe in the first place, can be used in reverse during retraction. When sucked into the needle, the porous red blood cells are compressed and release PBS. They shrink in size and allow the surrounding polymer structure to deform in order to fit through the needle opening. Inside the syringe, the red blood cells uptake PBS and swell to their original size, consequently regaining their initial shape.

Cytotoxicity is a crucial aspect for hydrogels used in medical applications. By retracting the hydrogel after it has completed its use in the body, possible cytotoxic materials that are incorporated in the hydrogel can completely be removed from the body. Such cytotoxic material can for example be magnetic heavy metals that are used for actuating the hydrogel in a magnetic field.

### Fabrication of magnetic soft robots and their magnetic response

After successful injections of the hydrogels, magnetic particles were added into the hydrogels, in order to actuate the hydrogels with an external homogeneous magnetic field.

Magnetic particles of 4-5 µm in diameter were integrated in the prepolymer solution in order to trap them in the polymer network of the hydrogel. Another possibility is to use magnetic nanoparticles, which can be integrated into the red blood cells together with drug molecules during the hypotonic-isotonic treatment. With an integration in the red blood cells, less polymerization issues are expected due to particles that block UV light. The hydrogels loaded with magnetic microparticles were still injectable and displayed a magnetic response when exposed to an external magnetic field. In fact, a magnetic gradient pulling could be observed when placing a hard ferromagnet next to the hydrogel. When including barium iron oxide particles into the prepolymer solution, the surface polymerized well, but underneath the prepolymer solution remained liquid, because of magnetic particles blocking the UV light. The issue could partly be solved by using semi-transparent stereolithography-printed molds (Durable material on Form 3, Formlabs, Somerville, MA) and turning the molds upside down during the polymerization process, to additionally expose the bottom of the mold to UV light. A certain improvement in the polymerization could be observed in this way. It is assumed that even better results can be achieved with more UV transparent materials, such as polydimethylsiloxane (PDMS). A reduction of the concentration of the magnetic microparticles from 5 wt% to 2 wt% resulted in fully polymerized hydrogels which still responded to magnetic fields.

### Example of a method for fabricating an injectable functional hydrogel

The preparation of the porous red blood cells (RBCs)
1. Add 0.5 mL of RBCs with 4.5 mL of 1X Phosphate Buffered Saline (PBS) into a 15 mL falcon tube
2. Mix the solution with a vortex mixer
3. Centrifuge the solution for 5 minutes at 2000 g and room temperature
4. Discard the supernatant
5. Dissolve the pellet in 5 mL of PBS
6. Repeat steps 2-5 two more times
7. Store the prepared solution in the fridge
8. Centrifuge the RBCs solution for 5 minutes at 2000 g and room temperature
9. Discard as much supernatant as possible
10. Add 1 mL of (1:1) (PBS:dH₂O) to the pellet
11. Incubate the solution for 15 minutes at 4°C
12. Centrifuge the solution for 5 minutes at 2000 g and room temperature
13. Discard as much supernatant as possible

The preparation of the prepolymer solution:
14. Add 37.5 mg of GelMA with 1 mL of PBS into a 1.5 mL Eppendorf tube
15. Vortex and sonicate the solution at 65 °C until the GelMA is completely dissolved
16. Add 37.5 mg of photoinitiator (LAP) to the solution in the dark
17. Wrap the Eppendorf with aluminium foil to protect the solution from light
18. Vortex and sonicate the solution at 65 °C until the LAP is completely dissolved
19. Add the GeIMA-LAP-PBS solution to the RBC pellet from step 13 and wrap it in aluminium foil
20. Mix the solution by pipetting and vortexing → No sonication, in order to not harm the red blood cells

The molding of the prepolymer solution and the fabrication of the injectable hydrogel networks:
21. Clean the molds with 70% ethanol and let them dry
22. Pour the prepolymer solution into the mold in the dark and avoid air bubbles
23. Expose the molds to UV radiation in 3 cycles of 20 seconds each
24. Cut out the polymerized scaffold from the mold
25. The injectable hydrogels are ready for the test

Figures 4a shows an inverted microscope image of the functional hydrogel material that has been fabricated according to the inventive method. Figures 4b and 4c show scanning electron microscope images of an exemplary hydrogel material that has also been fabricated according to the inventive method.

In the microscopic image shown in figure 4a, the individual porous red blood cells can just be recognized. With the increased magnification of figure 4b, the porous red blood cells that are integrated in the hydrogel material can be seen and the outer edges of their disc-like shape can be recognized. With the maximal magnification of figure 4c, not only the disc-shaped porous red blood cells 5 can easily be recognized, but also portions of the polymer 10 which surrounds and entraps the red blood cells 5 within the hydrogel material. Thus, by means of the scanning electron microscope, it is possible to identify the individual porous red blood cells 5 within the hydrogel material, and with a sufficient magnification, it is even possible to also recognize their characteristic outer shape which is similar to the one of natural, untreated red blood cells.

**LIST OF REFERENCE SIGNS**

| | | | |
|---|---|---|---|
| 1 | Prepolymer solution | 12 | Cannula |
| 2 | Prepolymer | 13 | Liquid |
| 3 | Regular red blood cell | 14 | Magnetic particles |
| 4 | Swollen red blood cell | 15 | Drug |
| 5 | Porous red blood cell | | |
| 6 | Pores | HT | Hypotonic treatment |
| 7 | Mold | IT | Isotonic treatment |
| 8 | Injectable hydrogel scaffold | C | Chemical treatment |
| 9 | Hydrogel material | UV | UV-exposure |
| 10 | Polymer | H | Heat treatment |
| 11 | Syringe | | |

## Claims

1. A method of preparing a functional hydrogel material (9) for injection into a human or animal body, the method comprising the step of polymerizing a prepolymer solution (1), in order to form the functional hydrogel material (9),
**characterized in that**
red blood cells (5) of which the cell membranes have been made porous are integrated into the prepolymer solution (1) before and/or during the polymerization.

2. The method as claimed in claim 1, wherein the red blood cells (5) are exposed to a hypotonic treatment (HT) and preferably to a subsequent isotonic treatment (IT), in order to make their cell membranes porous.

3. The method as claimed in claim 1 or 2, wherein the cytoplasm of the red blood cells (5) has been reduced by at least 50 wt%.

4. The method as claimed in one of the preceding claims, wherein UV-exposure (UV) is applied, in order to polymerize the prepolymer solution (1).

5. The method as claimed in one of the preceding claims, wherein the prepolymer solution (1) contains gelatin methacryloyl.

6. The method as claimed in claim 5, wherein the concentration of gelatin methacryloyl in the prepolymer solution (1) is between 2 wt% and 4 wt%.

7. The method as claimed in one of the preceding claims, wherein the number of the red blood cells (5) in the prepolymer solution (1) is between 7.5 and 10 billion/mL.

8. The method as claimed in one of the preceding claims, wherein pores (6) of the cell membranes of the red blood cells (5) have a diameter between 15 nm and 400 nm.

9. The method as claimed in one of the preceding claims, wherein the prepolymer solution (1) is polymerized in a mold (7).

10. The method as claimed in one of the preceding claims, wherein magnetic particles (14), a drug (15), a protein and/or cells are integrated into the functional hydrogel material (9) in addition to the red blood cells (5).

11. The method as claimed in one of the preceding claims, wherein the functional hydrogel material (9) is placed within an injection device, in particular a syringe (11), which comprises a cannula (12), a tube and/or a catheter, and which serves to inject or retract the functional hydrogel material (9) into or from the human or animal body through the cannula (12), the tube and/or the catheter.

12. The method according to claim 10, wherein, after placing the functional hydrogel material (9) within the injection device (11), the functional hydrogel material (9) is injected into the human or animal body through the cannula (12), the tube and/or the catheter.

13. The method as claimed in one of claims 10 or 11, wherein the functional hydrogel material (9) is placed within the injection device (11) by means of retracting the functional hydrogel material (9) from the body of the human or the animal through the cannula (12), the tube and/or the catheter.

14. The method as claimed in one of claims 10 to 12, wherein the red blood cells (5) contained in the functional hydrogel material (9) originate from the same human or animal, in which the functional hydrogel material (9) is to be injected or from which the functional hydrogel material (9) has been retracted.

15. The method as claimed in one of claims 10 to 13, wherein the red blood cells contained in the functional hydrogel material originate from a different human or animal, than the human or animal in which the functional hydrogel material is to be injected or from which the functional hydrogel material has been retracted.

16. The method according to one of claims 10 to 14, wherein the functional hydrogel material (9) has a structure which, in a relaxed state, covers a plane area that has a larger inner maximum diameter than the diameter of an inner lumen of the cannula (12), the tube and/or the catheter.

17. A functional hydrogel material (9) for injection into a human or animal body, in particular prepared by the method as claimed in one of the preceding claims, comprising a hydrogel network formed by a polymer (10);
**characterized in that**
red blood cells (5) of which the cell membranes have been made porous are integrated in the hydrogel network.
